Europäisches Patentamt

European Patent Office

Office européen des brevets

Publication number: **0 298 633**
**A2**

## EUROPEAN PATENT APPLICATION

(21) Application number: 88305771.3

(22) Date of filing: 24.06.88

(51) Int. Cl.⁴: **C07K 7/04 , A61K 37/02 , C07K 17/00 , C12N 15/00 , G01N 33/569 , A61K 39/42 , //A61K39/21,A61K39/385**

(30) Priority: 24.06.87 GB 8714802

(43) Date of publication of application: **11.01.89 Bulletin 89/02**

(84) Designated Contracting States: **ES GR**

(71) Applicant: **Proteus Biotechnology Limited Proteus House 48 Stockport Road Marple Cheshire SK6 6AB(GB)**

(72) Inventor: **Fishleigh, Robert V. 3 Ballbrook Avenue Didsbury Manchester(GB)** Inventor: **Robson, Barry 26 Jessop Drive Marple Stockport Cheshire(GB)**

(74) Representative: **Hildyard, Edward Martin et al Frank B. Dehn & Co. European Patent Attorneys Imperial House 15-19 Kingsway London WC2B 6UZ(GB)**

(54) Synthetic polypeptides.

(57) The invention relates to novel polypeptides. It particularly relates to polypeptides with a three-dimensional structure analogous to the three-dimensional structure of specific sites on the exterior surface of viral envelope proteins. It is of particular interest to the design of vaccines, diagnostics and other medical or scientific agents in relation to the Human Immunodeficiency Virus (HIV) as the causative agent of Acquired Immune Deficiency Syndrome (AIDS).

EP 0 298 633 A2

## Synthetic Polypeptides

The present invention relates to novel polypeptides. It particularly relates to polypeptides with a three-dimensional structure analogous to the three-dimensional structure of specific sites on the exterior surface of viral envelope proteins. It is of particular interest to the design of vaccines, diagnostics and other medical or scientific agents in relation to the Human Immunodeficiency Virus (HIV) as the causative agent of Acquired Immune Deficiency Syndrome (AIDS).

With the recent development of techniques for the rapid determination of protein and nucleic acid sequences there has come a need for computational and other methods to store and analyse such sequence data. Methods have been developed by this and other groups of workers for the prediction of protein secondary structures and the detection of homology between the sequences of different proteins. Computer graphics and other conformational calculation methods are rapidly advancing fields which can enable modelling of energetically preferred protein tertiary structures when only the primary protein structure i.e. the sequence of amino acid residues is known. Knowledge of the probable three-dimensional structures of proteins provides a large amount of information relating to the external surface structure of the protein. Such information enables the identification of particular regions of interest such as the active sites of enzymes i.e. substrate binding sites; hormone receptor and antibody binding sites of membrane proteins; and the antigenic determinants of a wide variety of different proteins.

A variety of techniques developed by ourselves now enable particular sites on the external surface of proteins to be identified and analysed in still greater detail. Such programs have been developed to predict polypeptide sequences calculated to have three-dimensional structures and/or chemical/electrostatic properties analogous to particular epitopic sites (regions which extend outwards from the three-dimensional surface structure) of proteins. At least some of such epitopic sites have been shown to correspond to the antigenic determinants of the proteins and hence the polypeptides calculated to be analogous are expected to have similar antigenic properties.

Any one epitopic site may comprise a continuous peptide sequence or may comprise a discontinuous determinant i.e. two or more sequences of amino acids which, whilst they may be widely separated in the primary structure of the protein, lie in close proximity in the tertiary protein structure and together constitute a single epitopic site. The predicted sequences may be variants of specific sections of the protein sequence or, particularly where they represent a discontinuous determinant, they need not bear a direct resemblance at a primary structural level. The predicted amino acid sequences and combinations of amino acid sequences are invariably much shorter than the amino acid sequences of the complete proteins and such polypeptides can be easily synthesised to provide a simple and effective route, using methods already well known in the art, to the production of peptide vaccines and antibodies. This approach also offers the possibility of basing a vaccine on some antigenic features common to a number of different strains of HIV, of which several have already been identified and more are expected to emerge from natural mutation and selection.

The HIV virus responsible for Acquired Immune Deficiency Syndrome (AIDS) is presently of great medical importance throughout the world and there is an urgent need for agents suitable for the scientific study, diagnosis, treatment and/or prevention of infection by the HIV virus. The amino acid sequences of the envelope protein of the HIV I and II viruses are now known (see, for example, Ratner, L. et al., Nature 313, 277 (1985); Meusing, M.A. et al., Nature 313, 450 (1985); Wain-Hobson, S. et al., Cell 40, 9 (1985)) but the successful production of vaccines, antibodies and/or specific agents for the treatment of HIV infection has not yet been achieved.

Thus, one aspect of our invention provides a synthetic polypeptide having at least one antigenic property of the envelope protein of at least one strain of Human Immunodeficiency Virus (HIV).

Another aspect of our invention provides a vaccine comprising at least one synthetic antigenic polypeptide effective to promote immunity against at least one strain of HIV.

A knowledge of the predicted secondary structure of the protein in question is of benefit in the design of peptide analogues of the continuous antigenic determinants, while a model for the three-dimensional structure of the protein is a prerequisite for the rational design of peptides corresponding to the non-continuous determinants. Some of the predicted polypeptides are rather short and correspond to predicted continuous antigenic determinants, and are likely to be most effective at raising neutralising antibody when linked to a suitable protein carrier molecule, although they may be effective on their own. Other peptides are rather larger and represent polypeptides designed to be structurally reminiscent of the three-dimensional conformation adopted by the equivalent portion of the native protein.

These latter polypeptides are thus designed to offer some of the non-continuous antigenic determinants present in the native protein. Such non-continuous epitopes are generally thought to comprise around two-thirds of all the antigenic determinants presented by proteins, and so peptide analogues of such predicted sites are likely to have a higher chance of successfully raising neutralising antibodies. By virtue of their larger size there is a greater probability that they may be effective at raising neutralising antibody on their own, although the possibility of coupling to a suitable protein carrier is nevertheless worthy of consideration and investigation. In all cases the polypeptides detailed here are very much shorter than the proteins of which they are a part, and in many cases some modification of the basic peptide may be required before it is fit for immunological usage.

It has been calculated that the following synthetic polypeptides have tertiary structures analogous to particular epitopic regions of the HIV I and/or HIV II envelope protein and are expected to show analogous antigenic properties and hence prove effective in raising antibodies that can specifically recognise the HIV viral particles. Such antibodies are expected to recognise epitopic sites which are similar in both HIV I and HIV II as well as analogous sites in other HIV variants such as HIV III which have not yet been fully characterised.

It will be appreciated that a random selection of segments from the primary protein structure is highly unlikely to result in the formation of a suitably antigenic analogue of the protein, since not only the relevant region of the amino acid sequence must be selected but also the correct length of peptide chain and its subsequent coupling to a carrier molecule are of vital importance. Only by our complex analyses of the protein sequence, which include secondary and tertiary structure modelling together with considerations of conserved regions of homology and variability between strains and of the sites of most frequent glycosylation, is it possible to design peptide analogues which preserve those features which our analyses have shown to be significant in determining the overall structure and specific surface characteristics of the native protein.

All the sequences are stated using the I.U.P.A.C. one-letter-code abbreviations for amino acid residues, defined as follows: G-Glycine, A-Alanine, V-Valine, L-Leucine, I-Isoleucine, S-Serine, T-Threonine, D-Aspartic acid, E-Glutamic acid, N-Asparagine, Q-Glutamine, K-Lysine, H-Histidine, R-Arginine, F-Phenylalanine, Y-Tyrosine, W-Tryptophan, C-Cysteine, M-Methionine and P-Proline.

The preferred polypeptide sequences were those chosen on the basis of their topographical similarity to at least one other antigenic determinant of the HIV envelope proteins. The antigenic properties of such peptides have been shown to raise antibodies which will interact with more than one antigenic site and hence are more likely to lead to an effective agent and/or vaccine against HIV.

The peptides most favoured on this basis were synthesised using either standard 9-fluorenyl-methoxycarbonyl (F-Moc) chemistry (see Kafiffaludy, L. & Schon, I., Synthesis $\underline{325}$ (1973); Atherton, E. & Shepphard, R.C., J.C.S. Chem. Com. $\underline{165}$ (1985)) or standard butyloxycarbonate (T-Boc) chemistry. The correctness of the structure and the level of purity was carefully checked, and particular attention was given to the correctness of internal disulphide bridging arrangements when present. In the present case various chromatographic analyses, including high performance liquid chromatography, and spectrographic analyses, including Raman spectroscopy, have been employed for this purpose.

Initially, two amino acid sequences:

(a) YYGVPVWKEATTTLFCA and

(b) KPCVKLTPLCV were selected because analysis revealed that these resemble a number of other sequence sites in the HIV envelope protein which were promising epitopes on the basis of our computational analysis. It was found that the envelope proteins can be partitioned into some five or six long segments with weak sequence homology, suggesting duplication of ancestral genes. However, detection of such similarity requires the application of the computer methods developed by ourselves since it is by no means obvious to the eye. Thus, for example, the reader may note the alignment of the first of these two peptides (a) with a sequence of a HIV-1 transmembrane segment (c) and a further sequence (d) defining another predicted antigenic determinant of HIV:

(a) YYGVPVW--KEATTTLFCA

(c) LLQLTVWGIKQLQARIL-A

(d) QLL-SGIVQQQNNLL The dashes indicate insertions introduced into the alignment in order to optimise sequence homology.

It has been found that linking the abovementioned sequences (a) and (b) together forms a new peptide which is itself topographically related to the natural segment of sequence commencing with peptide (c) above, as well as with sequences in other proteins which are not contiguous but are brought together by the spatial arrangment of the protein chain.

Thus, the present invention particularly provides the amino acid sequences:

(a) YYGVPVWKEATTTLFCA;

(b) KPCVKLTPLCV;

(c) LLQLTVWGIKQLQARILA; and

(d) QLLSGIVQQQNNLL.

The invention further provides the amino acid sequence:

(e) YYGVPVWKEATTTLFCASDQSLKPCVKLTPLCV. wherein the C-terminal of sequence (a) is linked via a connector peptide to the N-terminal of sequence (b). Preferably, the first two cysteine residues are linked to each other via an intramolecular disulphide bridge. The C-terminal dipeptide, CV, is not essential and need only be present, for example, if the sequence is to be linked through the cysteine side-chain to another molecule which may serve as a carrier. Thus, the invention further provides the amino acid sequence:

(e') YYGVPVWKEATTTLFCASDQSLKPCVKLTPL optionally containing an intramolecular disulphide bridge.

The peptide (e') was synthesised using T-Boc chemistry and the intramolecular disulphide bridge specifically formed between the two C residues. This peptide was used in an unconjugated form to raise antibodies in laboratory rabbits in conventional manner. The peptide stimulated a very strong antibody response in rabbits. Interaction was also observed between the antibodies raised and HIV protein and between HIV-positive sera and the synthetic peptide. This peptide is seen as a highly valuable tool for research studies where an agent which recognises a number of different HIV strains rather than one specific strain is highly desirable.

However, the topography of the above peptide may be modified to provide particularly preferred variants of peptide (e) or (e'). These include peptides wherein one or more C residues are each independently replaced by S residues and/or the DQ peptide is replaced by DGGGDQ or by another related sequence containing a greater or lesser number of G residues between the two D residues.

The following points were also considered in the production of preferred sequences:

(i) the loop stereochemistry implied by the choice of particular residues for disulphide bonding;

(ii) the solubility of the peptide (peptide (e') is only poorly soluble in aqueous solution);

(iii) the immunogenicity in the absence of a carrier; and

(iv) the ability to interact strongly with HIV-positive sera and to raise anti-HIV antibody.

These considerations allowed the stereochemistry of peptide (e) and of the derivative form (e') to be advantageously modified. Thus, it is preferred either to truncate peptide (e) such that the N-terminus commences with DQ:

(f) DQSLKPCVKLTPLCV or to delete the first C residue by replacing the triplet FCA with AA:

(g) YYGVPVWKEATTTLAASDQSLKPCVKLTPLCV. If the latter modification is made then VWK may optionally be replaced by VWGXK, wherein X represents any small hydrophobic residue such as V, L, A or I, preferably A. These modifications mean that the C residue in the triplet PCV no longer participates in an intramolecular disulphide bond and hence it is preferred either to replace the PCV by DQQ or to introduce a C residue to provide a new disulphide bond, for example by replacing KLTP by KLTCP.

The interaction with HIV-positive sera may be further enhanced by replacing the segment KLTCP by KLXCSSXXP, wherein the X residues may each independently represent any small hydrophobic residues such as V, L, A or I, preferably A, and one or more of the S residues may each independently be replaced by T residues. Furthermore, the region around the other C participating in the disulphide bond is preferably changed from KPCV to LGXWGCSG wherein X and S residues may each independently be replaced in the manner described above.

The following peptides are predicted to correspond to some of the continuous epitopic sites of the external and transmembrane glycoproteins of HIV and represent very specifically defined lengths and sequences of the native polypeptides:

(h) The polypeptide GEFLYC wherein the L is optionally replaced by F and which polypeptide sequence is optionally preceded by NCG or NCR and optionally followed by N;

(i) The polypeptide IKQIINAW wherein the A is optionally replaced by any residue and one or more I residues may each independently be replaced by F, said polypeptide being optionally preceded by PCR, PCH or PCK or chemically similar sequences, particularly those wherein the C residue is substituted as, for example, in PAR;

(j) The polypeptide PLGVAPTKAKR wherein the second A, the L and the V are each optionally replaced by any residue, and in particular wherein the second A is replaced by E, the L by I and/or the V by F;

(k) The polypeptide FLGFLAAAGSAMGAASMT wherein the first two A's, the fourth A, and the second M are each optionally replaced by any residue, and in particular wherein the first two A's may each independently be replaced by G or T, the fourth A by T and/or the second M by L or A;

(l) The polypeptide QARQLLSGIVQQQNNLL which .may optionally be preceded by H or a small hydrophobic residue and wherein the first A, the first S, and the second Q may each independently be replaced by any residue, and in particular by S, A, and/or T respectively, and the N residues may each independently be replaced by Q residues;

(m) The polypeptide QQXLLXLTVWGIK wherein X represents any residue, wherein I may be replaced by any residue, in particular by T and wherein the N-terminal QQ may optionally be deleted;

(n) The polypeptide QQEKNGGEL wherein one or more G residues may each independently be replaced by any other residue; and

(o) The polypeptide IPRRIRQGLEAALL wherein the first I and the first L may each independently be replaced by any residue, in particular V and/or A respectively and the A residues may each independently be replaced by any residue.

It is to be understood that any antigenically significant sub-fragment of the above-identified polypeptide sequences, and any variations thereof which maintain the general form and function of the polypeptides, are included within the scope of this invention. In particular, the substitution of any of the specific residues by residues having comparable physical properties such as, for example, are collected in the following groups: Group 1 -A, V, L, I, F, Y, W and M; Group 2 - S, T, N and Q; Group 3 - D and E; Group 4 - K, H and R; Group 5 -N and D; Group 6 - E and Q; Group 7 - G, A, P, S and T, are included.

The polypeptides are optionally linked to additional amino acids e.g. cysteine residues or to a conventional vaccine carrier, either at their C- or N-terminus or through residue sidechains, to render them optimal for their immunological function. The carrier-polypeptide link may involve the insertion of one or more residues, especially glycine, to form a bridge between the carrier and the polypeptide sequence. Suitable carriers include purified protein derivatives of tuberculin (PPD) and tetanus toxoid although the use of conventional non-protein carrier molecules is not precluded. When using PPD as a vaccine carrier, a higher titre of antibodies is achieved if the recipient of the polypeptide-PPD conjugate is already tuberculin sensitive, e.g. by virtue of an earlier BCG vaccination. In the UK and many other countries the population is routinely offered BCG vaccination and is therefore largely PPD-sensitive. Hence PPD is expected to be a preferred carrier for use in such countries.

The mode of coupling the polypeptide to the carrier will depend on the nature of the materials to be coupled. For example, a lysine residue in the carrier may be coupled to a C-terminal or other cysteine residue in the polypeptide by treatment with N-$\gamma$-maleimidobutyryloxysuccinimide. Other coupling reactions and reagents have been described in the literature.

The HIV envelope protein (as the gene product) is _in vivo_ proteolytically cleaved into two polypeptide fragments, each of which exists as a separate protein in the virus. The smallest of these two probably corresponds to a transmembrane protein. Synthetic polypeptide sequences corresponding to parts of this transmembrane polypeptide are described in sections (k) - (o) and it is preferred that these polypeptides should be linked to a carrier to enhance their antigenic properties as is also the case for all the preceding peptides. It is particularly preferred for polypeptides (k) - (n) to be linked to a carrier at their C-terminus. Polypeptide (o) corresponds to a site involving the C-terminus end of the native envelope protein and as such it is highly preferred to link a carrier to the N-terminus of this peptide in order to leave the C-terminus-end free.

It is to be understood that the polypeptides of the invention may be synthesised by any conventional method, either directly using manual or automated peptide synthesis techniques, or indirectly by RNA or DNA synthesis and conventional techniques of genetic engineering. Such techniques include the production of hybrid proteins containing one or more of the above peptides inserted into another polypeptide sequence.

Another aspect of the present invention therefore provides DNA molecules coding for at least one synthetic antigenic polypeptide as described herein, preferably incorporated into a suitable expression vector replicable in microorganisms or in mammalian cells. The DNA may also appear as part of the DNA sequence for a longer product e.g. the polypeptides may be expressed as parts of other proteins into which they have been inserted by genetic engineering.

The antigenic polypeptides are of use, either alone or linked to an appropriate carrier as:

(a) peptide vaccines, for use to prevent infection by one or more strains of HIV;

(b) as antigenic agents for the generation of antibodies, either monoclonal or polyclonal, by immunisation of an appropriate animal, such antibodies being of use both for the scientific study of the HIV virus and as diagnostic agents;

(c) the treatment of HIV infections, either by displacing the binding of the HIV virus to human or animal cells or by disturbing the three-dimensional organisation of the virus _in vivo_ or _in vitro_; as well as aiding the scientific study of HIV viruses _in vitro_.

The preparation of antibodies, monoclonal or polyclonal, which specifically bind to a synthetic polypeptide according to the present invention may be carried out by conventional means and such antibodies are intended to form part of the invention. The antibodies according to the invention are of use in a method of diagnosing mammalian HIV infection which comprises incubating a sample of tissue or body fluid of a mammal with an effective amount of an antibody as claimed in claim 22 and determining whether, and if desired the extent to which and/or rate at which, cross-reaction between said sample and said antibody occurs. A diagnostic kit which contains at least one of said antibodies is also provided.

A further aspect of the invention provides synthetic polypeptides for use in therapy or prophylaxis of mammalian HIV infection and/or stimulating the mammalian immune system and/or blocking the cellular receptors for the HIV virus and for the preparation of medicaments suitable for such uses. Also included are pharmaceutical compositions containing, as active ingredient, at least one polypeptide or polypeptide-carrier conjugate as described herein in association with one or more pharmaceutically acceptable adjuvants, carriers and/or excipients. The compositions may be formulated for oral, rectal, nasal or especially for parenteral administration.

The invention further provides a method of therapy or prophylaxis of mammalian HIV infection and/or of stimulating the mammalian immune system and/or of blocking the cellular receptors for the HIV virus, which comprises administering an effective amount of a polypeptide as hereinbefore defined.

The following examples are intended to illustrate the invention and are not limiting in any way.

## Example 1

Peptide (e') was synthesised using T-Boc chemistry with 10% aqueous acetic acid as solvent.

The peptide (6.7 mg per immunisation) was then injected intradermally into rabbits using aluminium hydroxide as the adjuvant. Rabbit sera was taken 7 days after the single immunisation and the cross-reaction between peptide (e') and the rabbit sera was assayed.

A number of serological assay conditions were tested and it was noted that tests requiring adsorption of the antigen onto a surface such as red blood cells (haemagglutination assay), latex particles (latex agglutination assay) and enzyme immunoassay on plastic (ELISA) were generally unsuccessful. This was attributed to the acidity of the antigen preparation (synthesised in 10% acetic acid) since all the above mentioned tests require alkaline absorption conditions.

However, serological assay techniques successfully used included gel diffusion, in which antigen and antibody diffuse towards each other through an agar gel, and thin layer immunoassay on, for example, nitrocellulose or PVDF membranes (Dot ELISA). The results are shown in Table 1 below.

**Table 1:** Cross-reaction between peptide (e') and rabbit serum

| Serological assay technique | Cross-reaction | |
|---|---|---|
| | Control serum | Serum taken 7 days after immunisation |
| Gel diffusion | − | +++ |
| Dot ELISA | − | +++ |

NB    Cross-reaction was scored as:

           –   no cross-reaction

           +   weak cross-reaction

           ++  average cross-reaction

           +++ strong cross-reaction

Preliminary results under similar conditions indicated a cross-reaction between the immunised rabbit sera and extracts from a commercially available HIV protein preparation.

**Claims**

1. A synthetic polypeptide having at least one antigenic property of the envelope protein of at least one strain of Human Immunodeficiency Virus (HIV).

2. A synthetic polypeptide as claimed in claim 1 having the amino acid sequence:

(a) YYGVPVWKEATTTLFCA;

(b) KPCVKLTPLCV;

(c) LLQLTVWGIKQLQARILA; or

(d) QLLSGIVQQQNNLL.

3. A synthetic polypeptide as claimed in claim 1 having the amino acid sequence:

(e) YYGVPVWKEATTTLFCASDQSLKPCVKLTPLCV or

(e') YYGVPVWKEATTTLFCASDQSLKPCVKLTPL

wherein optionally:

i) the two cysteine residues closest to the N-terminal are linked to each other via an intramolecular disulphide bridge; and/or

ii) one or more of the C residues are each independently replaced by S residues; and/or

iii) the DQ peptide is replaced by DGGGDQ or by another related sequence containing a greater or lesser number of G residues between the two D residues; and/or

iv) where at least two C residues are present in said polypeptide, two C residues are linked to each other via an intramolecular disulphide bridge.

4. A synthetic polypeptide as claimed in claim 1 having the amino acid sequence:

(f) DQSLKPCVKLTPLCV or

(g) YYGVPVWKEATTTLAASDQSLKPCVKLTPLCV

wherein optionally:

i) VWK is replaced by VWGXK wherein X represents any small hydrophobic residue; and/or

ii) PCV is replaced by DQQ; or

iii) an additional C residue is introduced into the amino acid sequence and an intramolecular disulphide bridge links the two C residues closest to the N-terminal and optionally KLTP is replaced by KLTCP or KLXCSSXXP and/or KPCV is replaced by LGXWGCSG, wherein the X residues may each independently represent any small hydrophobic residue and one or more of the S residues are each independently replaced by T residues.

5. A synthetic polypeptide as claimed in claim 4 wherein X represents a V, L, A or I residue.

6. A synthetic polypeptide as claimed in claim 5 wherein X represents an A residue.

7. A synthetic polypeptide as claimed in claim 1 having the amino acid sequence:

(h) GEFLYC wherein the L is optionally replaced by F and which polypeptide sequence is optionally preceded by NCG or NCR and optionally followed by N;

(i) IKQIINAW wherein the A is optionally replaced by any residue and one or more I residues may each independently be replaced by F, said polypeptide being optionally preceded by PCR, PCH or PCK or chemically similar sequences;

(j) PLGVAPTKAKR wherein the second A, the L and the V are each optionally replaced by any residue;

(k) FLGFLAAAGSAMGAASMT wherein the first two A's, the fourth A, and the second M are each optionally replaced by any residue;

(l) QARQLLSGIVQQQNNLL which may optionally be preceded by H or a small hydrophobic residue wherein the first A, the first S, and the second Q may each independently be replaced by any residue and the N residues may each independently be replaced by Q residues;

(m) QQXLLXLTVWGIK wherein X represents any residue, wherein I may be replaced by any residue and wherein the N-terminal QQ may optionally be deleted;

(n) QQEKNGGEL wherein one or more G residues may each independently be replaced by any other residue; or

(o) IPRRIRQGLEAALL wherein the first I, the first L and the A residues may each independently be replaced by any residue.

8. A synthetic polypeptide as claimed in claim 7 having the amino acid sequence:

(i) wherein the C residue in the leader sequence is substituted as, for example, in PAR;

(j) wherein the second A is replaced by E, the L by I and/or the V by F;

(k) wherein the first two A's may each independently be replaced by G or T, the fourth A by T and/or the second M by L or A;

(l) wherein the first A, the first S and the second Q may each independently be replaced by S, A and/or T respectively;

(m) wherein I is replaced by T; or

(o) wherein the first I and the first L may each independently be replaced by V and/or A respectively.

9. A synthetic polypeptide as claimed in claim 1 which comprises an antigenically significant subfragment or variant of a polypeptide as claimed in any one of claims 2 to 8.

10. A synthetic polypeptide as claimed in any one of claims 1 to 9 linked, optionally via one or more additional amino acid residues, to a vaccine carrier.

11. A vaccine comprising at least one synthetic polypeptide as claimed in any one of claims 1 to 10 effective to promote immunity against at least one strain of HIV.

12. A DNA molecule coding for at least one synthetic polypeptide as claimed in any one of claims 1 to 10.

13. A pharmaceutical composition containing, as active ingredient, at least one polypeptide as claimed in any one of claims 1 to 10 in association with one or more pharmaceutically acceptable adjuvants, carriers and/or excipients.

14. Use of a synthetic polypeptide as claimed in any one of claims 1 to 10 for the preparation of a medicament for the therapeutic or prophylactic treatment of mammalian HIV infection and/or for stimulating the mammalian immune system and/or blocking the cellular receptors for the HIV virus.

15. A method of therapy or prophylaxis of mammalian HIV infection and/or of stimulating the mammalian immune system and/or of blocking the cellular receptors for the HIV virus, which comprises administering an effective amount of a polypeptide as claimed in any one of claims 1 to 10.

16. An antibody, monoclonal or polyclonal, which specifically binds to a synthetic polypeptide as claimed in any one of claims 1 to 10.

17. A diagnostic kit for detecting HIV which contains at least one antibody as claimed in claim 16.

18. A method of diagnosing mammalian HIV infection which comprises incubating a sample of tissue or body fluid of a mammal with an effective amount of an antibody as claimed in claim 16 and determining whether, and if desired the extent to which and/or rate at which, cross-reaction between said sample and said antibody occurs.